(19) Europäisches Patentamt
European Patent Office
Office européen des brevets

(11) **EP 1 778 163 B1**

(12) **EUROPEAN PATENT SPECIFICATION**

(45) Date of publication and mention
of the grant of the patent:
**12.11.2014 Bulletin 2014/46**

(21) Application number: **05774832.9**

(22) Date of filing: **17.08.2005**

(51) Int Cl.:
*A61K 6/06* *(2006.01)*          *A61L 27/10* *(2006.01)*
*A61L 27/42* *(2006.01)*         *C04B 28/02* *(2006.01)*
*C04B 28/06* *(2006.01)*         *C04B 28/34* *(2006.01)*

(86) International application number:
**PCT/SE2005/001215**

(87) International publication number:
**WO 2006/019355 (23.02.2006 Gazette 2006/08)**

(54) **CHEMICALLY BONDED CERAMIC MATERIAL**

CHEMISCH GEBUNDENES KERAMIKMATERIAL

MATERIAU CERAMIQUE LIE CHIMIQUEMENT

(84) Designated Contracting States:
**AT BE BG CH CY CZ DE DK EE ES FI FR GB GR HU IE IS IT LI LT LU LV MC NL PL PT RO SE SI SK TR**

(30) Priority: **18.08.2004 SE 0402044**

(43) Date of publication of application:
**02.05.2007 Bulletin 2007/18**

(73) Proprietor: **Doxa AB**
**754 51 Uppsala (SE)**

(72) Inventors:
• **ENGQVIST, Håkan**
**S-741 44 Knivsta (SE)**
• **HERMANSSON, Leif**
**S-260 42 Mölle (SE)**

• **LÖÖF, Jesper**
**S-754 42 Uppsala (SE)**
• **SPENGLER, Håkan**
**S-753 34 Uppsala (SE)**

(74) Representative: **Brann AB**
**P.O. Box 12246**
**102 26 Stockholm (SE)**

(56) References cited:
**EP-A2- 0 250 120          WO-A1-01/76535**
**WO-A1-90/11066          WO-A1-03/041662**
**WO-A1-2004/000240          WO-A1-2004/000240**
**US-A- 4 804 643          US-A- 4 804 643**
**US-A- 5 055 430**

**Description**

**Field of the invention**

The present invention relates to a system for chemically bonded ceramic (CBC) materials, and a composite biomaterial for preferably dental and/or orthopaedic applications. The CBC-system includes a binding (chemical cement) phase and inert phases with specified geometry providing the biomaterial defect tolerance through an improved fracture toughness. The invention also relates to the powdered materials and the hydration liquid, respectively, as well as the formed ceramic material.

**Background art**

For materials to be used as filling materials, dental fillings or bone void fillers, which have to interact with human tissue it is advantageous to make the biomaterials as biocompatible and bioactive as possible. These and other properties required for dental and orthopaedic materials are easy handling, good mouldability, hardening and curing within a short time period to make the material useful as soon as possible, high hardness and strength, corrosion resistant, ability to close gaps between the injected material and the host tissue and exhibit radio opacity. These as well as other property aspects of chemically bonded ceramics (initial moulding ability, initial strength, heat evolved and early colour/transmittance development as well as high general strength), are dealt with in SE 463 493, SE 502 987, SE 519 990 , SE 519 991, SE 522 510, SE 522 511, SE 522 512 SE 523 671, SE Patent application 0203910-5, approved 30 June 2004, WO 00/21489, WO 01/76534 and WO 01/76535.

**Brief description of the invention**

The present invention relates to binding systems of the hydrating cement system type, in particular cement-based systems that comprise chemically bonded ceramics in the group that consists of aluminates, silicates, phosphates, carbonates, sulphates and combinations thereof, having calcium as the major cation, and in addition to the said system at least one second phase contributing to an increased defect tolerance. The invention has been especially developed for biomaterials for dental and orthopaedic applications, but can also be used as filler materials in industrial applications in electronics, micro-mechanics etc., or in the construction field.

The present invention specifically relates to the problems of remaining larger defects originating from pores, bubbles, insufficient mixing, a somewhat in-diligent handling, larger in-homogeneities origination from the raw materials, non-dispersed regions etc. These defects often determine the actual strength of the material and not the general microstructure of the same material. To assure the material some kind of threshold value as far as strength is concerned, it is important to find a means of demasking/removing the influence from these defects. To avoid these defects or to remove them totally by even more careful raw materials handling, develop operator's skill etc. are probably not doable. The present invention provides a tool that diminishes the harmful effect of these occasionally occurring defects by hindering or reducing the stress concentration around these defects. This is achieved by introducing fibers and (whisker or platelets) of specified sizes related to the defect size of possible defects discussed above. The fiber size is chosen so as to have the ability of shielding the defect but at the same time not working as a new defect. The proper choice of sizes is related to the basic fracture mechanics equation, described in more details below.

**Detailed description of the invention**

As distinguished from the survey article on strong and safe brittle ceramics "Strong and safe brittle ceramics - Materials aspects of design with structural ceramics", by L. Hermansson", in Advanced Ceramics for Structural and Tribological Applications, ed. Hawthorne and Troczynski, 1995, the present invention does not deal with the general microstructure of a material and the related strength, but with the practical strength of a material with remaining un-purposely introduced larger inhomogeneities. From the basic fracture mechanics equation

$$K_{IC} = Y\ \sigma\ c^{1/2},$$

where
$K_{IC}$ = fracture toughness in $MPam^{1/2}$
$\sigma$ = actual strength in MPa
c = defect size in meter

Y = position and shape factor, theoretically between 1.12 and 1.98, often around 1.7

the relationship between possible strength levels and defect sizes can be understood. For chemically bonded ceramics and biomaterials used for dental and orthopaedic applications the $K_{IC}$ - values are in the interval of 0.25 - 1.5 MPam$^{1/2}$. In example 1 the size of defects controlling the strength at different fracture toughnesses is presented in detail. The defect size from handling and arbitrary sources are often in the range of 20-80 $\mu$m. This indicates that the area to be shielded should be in the same range. The present invention addresses these issues.

Another aspect can be understood from the level of $K_{IC}$-values. In materials for use as dental filling materials or as bone void fillers, strength levels of more than 50 MPa are required with respect to bending strength. This also indicates that shield materials need to be 20-80 $\mu$m in length. Fibers of a length above this interval will need too high fracture toughness values to be used, or will constitute strength-controlling defects.

Accordingly, the present invention aims at providing a system for materials, preferably biomaterials, having a second phase as a shield or barrier with a length of at least 25 $\mu$m but not more than 80 $\mu$m.

The use of fibers and similar shield materials in the range of 25-80 $\mu$m does not exclude the use of fibers or whiskers with different sizes, especially not those with a length < 25 $\mu$m. However, the range of 25-80 $\mu$m is the crucial range for threshold values based on the risk of fractures for biomaterials.

These and other objectives are attained by the system, the powdered material, the hydration liquid and the ceramic material according to the invention, as defined in the claims.

Thus, CBC-materials with the binding phases mentioned and the shield-giving additives function so as to achieve high performance features related to biomechanical properties.

The binding particles in the present invention are composed of chemically bonded ceramics, preferably Ca-aluminates, preferably CA = $(CaO)(Al_2O_3)$, $C_{12}A_7 = (CaO)_{12}(Al_2O_3)_7)$ and $C_3A = (CaO)_3(Al_2O_3)$, and in addition inert phases including glass, e.g. a phosphorous-containing glass. The inert filler (stable glass particles or oxides) is essential for the general end-product microstructure. Its effect concerns a lowered expansion, increased radio-opacity and favoured mechanical properties, especially hardness and the general microstructurally related strength.

Concerning calcium aluminate phases it is preferable to use CA, $C_{12}A_7$ and $C_3A$, which yield good initial strength. The addition of an accelerator is dependant upon the selection of the Ca-aluminate phase. Low concentrations of lithium ions increase the reaction rate for CA. For $C_{12}A_7$ and $C_3A$ the effect of an accelerator is more complex.

It is preferred that the system comprises inert dental glass, as an additive in the powdered material, preferably at a content of 5-45 weight-%, more preferably 15-35 %. The particle size is critical in establishing high homogeneity. It is preferred that the mean particle size is 0.1-5 $\mu$m, more preferably 0.2-2 $\mu$m, and most preferably 0.3-0.7 $\mu$m. The glass may contain low amounts of less stable glass or reactive glass, preferably below 5 % of the glass content. These glasses can preferably contain fluorine and phosphorus to yield fluoride and phosphate ions, which contribute to F-apatite formation.

According to one aspect of the invention the inert filler particles are composed of prehydrated chemically bonded ceramics of the same composition or similar as that of the main binding phase. This improves the homogeneity of the microstructure and enhances the binding between reacting chemically bonded ceramics and the filler material.

According to another aspect of the present invention an additional system is included to improve the closure of pores initially, namely by introducing a system that works by pure water up-take, e.g. the semi-hydrate of $CaSO_4$, gypsum. And a further system to solidify the total system initially, the combination of phosphoric acid and zinc oxide-forming Zn-phosphate. These phases will not contribute to the long-term properties but will enhance the initial pore closure and initial strength.

The system and material according to the invention have the advantages compared to systems/materials such as glass ionomer cements and pure Ca-aluminate based systems or monomer based filling materials, that it maintains its bioactivity, that it has improved initial strength and that it has long time stability regarding both dimensional aspects, strength and minimised deterioration and improved fracture toughness and able of shielding sparsely occurring large defect, making the materials according to the invention robust and defect tolerant. The introduction of glass fibers according to present invention reduces the geometrical change during curing to a minimum. The viscosity of the material can be controlled within wide frames, upon initial mixing of the powdered material and the hydration liquid, from moist granules to an injectable slurry. However it is preferable to decrease the water to cement (w/c) ratio as much as possible leaving an appropriate viscosity for given application. The w/c ratio should be < 0.50, more preferably within the interval of 0.30-0.40. This is accomplished according to the present invention by using dispersing agents, preferably salts of polyacrylic acids.

EXAMPLE 1

In the table below is illustrated how the strength-controlling defect size is related to the actual strength for given fracture toughness levels in the interval 0.50-1.25 Mpam$^{1/2}$. Y is 1.98.

| $K_{IC}$ - MPam$^{1/2}$ | Tolerable defect size in $\mu$m at 25 MPa | Tolerable defect size in $\mu$m at 50 MPa | Tolerable defect size in $\mu$m at 75 MPa | Tolerable defect size in $\mu$m at 100 MPa |
|---|---|---|---|---|
| 0.5 | 100 | **25** | 11 | 6 |
| 0.75 | 225 | 55 | **25** | 14 |
| 1.0 | 400 | 100 | 44 | **25** |
| 1.25 | 625 | 156 | 104 | 39 |

As is clearly seen defects larger than 25 $\mu$m should be removed or shielded to avoid fracture at the given stress levels for typical fracture toughness levels for chemically bonded ceramics. For a Y-value of 1.7 the limit is approximately 30 $\mu$m.

EXAMPLE 2

Tests were performed to investigate the influence of shielding additives on the obtained flexural strength of material. The fracture toughness was also measured.

Description of raw materials

Calcium aluminate ($(CaO)_3(Al_2O_3)$, $(CaO)(Al_2O_3)$, $(CaO)_{12}(Al_2O_3)_7$) dental glass filler (Schott), poly acid (Na-PAMA = poly(acrylic-co-maleic acid) sodium salt Mw=50,000 and glass fibers G1, G2 at different contents. Glass fiber G1 had an average length:diameter ratio of 20, with an average diameter of 3 $\mu$m. Glass fiber G2 had an average length:diameter ratio of 40, with an average diameter of 1.5 $\mu$m. The fibers are based on Ca-silicate glasses. As reference material a commercial glass ionomer cement (Dentsply) was used.

Description of materials

Calcium aluminate was mixed with dental glass and poly(acrylic-co-maleic acid) sodium salt and the glass fibers. The calcium aluminate phases were synthesised via a sintering process where first CaO and $Al_2O_3$ were mixed to the desired composition and then sintered at elevated temperature for 6 hours. The formed calcium aluminate lumps were crushed and jet-milled to a mean grain size of 1.5 $\mu$m and a maximum grain size of 9 $\mu$m. The dental glass, calcium aluminate and poly acids were mixed with acetone and $Si_3N_4$ marbles for 14 hours to obtain the desired homogeneity. Formulations were made according to (in wt.%):

| Formulation | Calcium aluminate phase | Inert glass | Na-PAMA Mw 5000 | Glass fiber G1 | Glass fiber G2 |
|---|---|---|---|---|---|
| 1 | $(CaO)(Al_2O_3)$ 60 | 38 | 2 | 0 | 0 |
| 2 | $(CaO)(Al_2O_3)$ 60 | 30 | 2 | 8 | 0 |
| 3 | $(CaO)(Al_2O_3)$ 60 | 30 | 2 | 0 | 8 |
| 4 | $(CaO)(Al_2O_3)$ 60 | 20 | 2 | 18 | 0 |
| 5 | $(CaO)(Al_2O_3)$ 60 | 20 | 2 | 0 | 18 |
| 6 | $(CaO)(Al_2O_3)$ / $(CaO)_{12}(Al_2O_3)_7$ mineral mixture of 80/20 and 60 in total | 23 | 2 | 15 | 0 |
| 7 | $(CaO)(Al_2O_3)$ / $(CaO)_{12}(Al_2O_3)_7$ mineral mixture of 50/50 and 60 in total | 23 | 2 | 0 | 15 |
| Ref-material | Glassionomer cement | | | | |

The formulations were placed in 5 ml jars and wet with liquid and blended in a "rot-mix" (3M ESPE) for 15 seconds followed by centrifugation for 3 seconds. In addition 18 mM of LiCl was added to further increase the hydration speed.

Description of tests

The flexural strength was measured according to ASTM F-394, $K_{IC}$ according to the single edge notch technique, and hardness according to Vickers indentation at 100 g for the eight formulations. All samples were stored at 37°C in phosphate buffer system 0.02 M (pH 7.4) for one week before testing.

Results

The results from the testing are presented in the table below.

| Formulation | Flexural Strength MPa | KIC MPam1/2 | Hardness Vicker indentation at 100 g |
|---|---|---|---|
| 1 | 65 | 0.61 | 105 |
| 2 | 72 | 0.78 | 110 |
| 3 | 85 | 0.88 | 118 |
| 4 | 85 | 0.98 | 115 |
| 5 | 88 | 1.03 | 115 |
| 6 | 90 | 1.05 | 114 |
| 7 | 86 | 1.00 | 116 |
| Ref-material | 60 | 0.50 | 70 |

The glass fiber addition increases the $K_{IC}$ essentially above that of the glass ionomer cement. The glass fiber with the larger surface contact area (G2) has a somewhat higher fracture toughness than that of the G1 formulation. The strength of the glass fiber-reinforced material is above 80 MPa.
The results show the fiber-reinforced material to have the potential to meet general mechanical requirements put on biomaterials for injectable dental and orthopaedic applications.
The system is not restricted to biomaterials application, and can favourably be used for industrial applications in electronics, micro-mechanics etc or in the construction field.

**Claims**

1. A chemically bonded ceramic biomaterial system for dental and orthopaedic applications having a fracture toughness exceeding 0.5 MPam$^{1/2}$ wherein the chemically bonded binding phase is composed of an aluminate based, and/or silicate based and/or phosphate-based cement, **characterised in that** the system contains a defect shielding agent, comprising fibers and/or whiskers and/or platelets, in an amount exceeding 5 vol-% and of an average length of 30 $\mu$m to 80 $\mu$m.

2. The system according to claim 1, **characterised in that** the defect shielding agent is contained in an amount of 7-15 vol-%.

3. The system according to any one of the preceding claims, **characterised in that** the fiber length to diameter ratio is < 180, more preferably < 60 and most preferably within the interval 10-40.

4. The system according to any one of the preceding claims, **characterised in that** the fiber diameter is < 5 $\mu$m, more preferably < 3 $\mu$m and most preferably within the interval 0.5-1.5 $\mu$m.

5. The system according to any one of the preceding claims, **characterised in that** the whisker or platelet length to diameter ratio is < 100, more preferably < 50.

6. The system according to any one of the preceding claims, **characterised in that** the chemically bonded binding phase is composed of an aluminate based, and/or silicate based and/or phosphate-based cement with Ca as cation.

7. The system according to claim 6, **characterised in that** the material also contains a second organic chemically based material, preferably a poly acrylic based material.

8. The system according to any one of the preceding claims, **characterised in that** the water to cement ratio is < 0.50, more preferably within the interval 0.30-0.40.

9. The system according to any one of the preceding claims, **characterised in that** the fiber is composed of an inorganic glass.

10. The system according to claim 9, **characterised in that** the glass fiber is based on a Ca-silicate.

11. The system according to any one of the preceding claims, **characterised in that** the whiskers and/or platelets are composed of inorganic mineral phases, preferably a Ca-silicate based mineral.

12. The system according to claim 1, **characterised in that** the material has a $K_{IC}$-value after curing > 7 days in the interval 0.8-1.2 $MPam^{1/2}$.

13. The system according to claim 1, **characterised in that** the material has a dimensional change of < 0.2 linear %, and/or an expansion pressure during hardening of < 3 MPa.

**Patentansprüche**

1. Ein chemisch gebundenes keramisches Biomaterialsystem für dentale und orthopädische Anwendungen mit einer Bruchzähigkeit von über 0,5 $MPam^{1/2}$, wobei die chemisch gebundene Bindungsphase aus einem Zement auf einer Aluminatbasis und/oder Silikatbasis und/oder Phosphatbasis zusammengesetzt ist, **dadurch gekennzeichnet, dass** das System ein Defekt abschirmendes Mittel, das Fasern und/oder Whisker und/oder Plättchen umfasst, in einer Menge von mehr als 5 Vol.-% und mit einer durchschnittlichen Länge von 30 µm bis 80 µm enthält.

2. Das System nach Anspruch 1, **dadurch gekennzeichnet, dass** das Defekt abschirmende Mittel in einer Menge von 7-15 Vol.-% enthalten ist.

3. Das System nach einem der vorhergehenden Ansprüche, **dadurch gekennzeichnet, dass** das Verhältnis von Faserlänge zu Durchmesser < 180, stärker bevorzugt < 60 beträgt und am meisten bevorzugt im Bereich von 10 - 40 liegt.

4. Das System nach einem der vorhergehenden Ansprüche, **dadurch gekennzeichnet, dass** der Faserdurchmesser < 5 µm, stärker bevorzugt < 3 µm beträgt und am meisten bevorzugt im Bereich von 0,5-1,5 µm liegt.

5. Das System nach einem der vorhergehenden Ansprüche, **dadurch gekennzeichnet, dass** das Verhältnis der Whisker- oder Plättchenlänge zum Durchmesser < 100, stärker bevorzugt < 50 beträgt.

6. Das System nach einem der vorhergehenden Ansprüche, **dadurch gekennzeichnet, dass** die chemisch gebundene Bindungsphase aus einem Zement auf einer Aluminatbasis und/oder Silikatbasis und/oder Phosphatbasis mit Ca als Kation zusammengesetzt ist.

7. Das System nach Anspruch 6, **dadurch gekennzeichnet, dass** das Material auch ein zweites organisches Material auf chemischer Basis, vorzugsweise ein Material auf Polyacrylsäure-Basis, enthält.

8. Das System nach einem der vorhergehenden Ansprüche, **dadurch gekennzeichnet, dass** das Verhältnis von Wasser zu Zement < 0,50 beträgt, stärker bevorzugt im Bereich von 0,30-0,40 liegt.

9. Das System nach einem der vorhergehenden Ansprüche, **dadurch gekennzeichnet, dass** die Faser aus einem anorganischen Glas zusammengesetzt ist.

10. Das System nach Anspruch 9, **dadurch gekennzeichnet, dass** die Glasfaser auf einem Ca-Silikat basiert.

11. Das System nach einem der vorhergehenden Ansprüche, **dadurch gekennzeichnet, dass** die Whisker und/oder Plättchen aus anorganischen Mineralphasen, vorzugsweise einem Mineral auf Ca-Silikat-Basis, zusammengesetzt sind.

**12.** Das System nach Anspruch 1, **dadurch gekennzeichnet, dass** das Material einen $K_{IC}$-Wert nach dem Härten > 7 Tage im Bereich von 0,8-1,2 MPam$^{1/2}$ aufweist.

**13.** Das System nach Anspruch 1, **dadurch gekennzeichnet, dass** das Material eine Dimensionsänderung von < 0,2 linearen % und/oder einen Expansionsdruck während des Härtens von < 3 MPa aufweist.


**Revendications**

**1.** Système de biomatériau céramique chimiquement lié destiné à des applications dentaires et orthopédiques ayant une ténacité supérieure à 0,5 MPam$^{1/2}$, dans lequel la phase de liaison chimiquement liée est constituée d'un ciment à base d'aluminate et/ou à base de silicate et/ou à base de phosphate, **caractérisé en ce que** le système contient un agent de protection contre les défauts, comprenant des fibres et/ou des trichites et/ou des plaquettes, en une quantité supérieure à 5 % en volume et d'une longueur moyenne de 30 $\mu$m à 80 $\mu$m.

**2.** Système selon la revendication 1, **caractérisé en ce que** l'agent de protection contre les défauts est contenu en une quantité de 7 à 15 % en volume.

**3.** Système selon l'une quelconque des revendications précédentes, **caractérisé en ce que** le rapport de la longueur au diamètre des fibres est < 180, mieux encore < 60 et tout particulièrement dans l'intervalle de 10 à 40.

**4.** Système selon l'une quelconque des revendications précédentes, **caractérisé en ce que** le diamètre des fibres est < 5 $\mu$m, mieux encore < 3 $\mu$m et tout particulièrement dans l'intervalle de 0,5 à 1,5 $\mu$m.

**5.** Système selon l'une quelconque des revendications précédentes, **caractérisé en ce que** le rapport de la longueur au diamètre des trichites ou des plaquettes est < 100, mieux encore < 50.

**6.** Système selon l'une quelconque des revendications précédentes, **caractérisé en ce que** la phase de liaison chimiquement liée est constituée d'un ciment à base d'aluminate et/ou à base de silicate et/ou à base de phosphate avec du Ca comme cation.

**7.** Système selon la revendication 6, **caractérisé en ce que** le matériau contient aussi un second matériau à base d'un composé chimique organique, de préférence un matériau à base d'un composé polyacrylique.

**8.** Système selon l'une quelconque des revendications précédentes, **caractérisé en ce que** le rapport de l'eau au ciment est < 0,50, mieux encore dans l'intervalle de 0,30 à 0,40.

**9.** Système selon l'une quelconque des revendications précédentes, **caractérisé en ce que** la fibre est constituée de verre inorganique.

**10.** Système selon la revendication 9, **caractérisé en ce que** la fibre de verre est à base de silicate de Ca.

**11.** Système selon l'une quelconque des revendications précédentes, **caractérisé en ce que** les trichites et/ou les plaquettes sont constituées de phases minérales inorganiques, de préférence un minéral à base de silicate de Ca.

**12.** Système selon la revendication 1, **caractérisé en ce que** le matériau a une valeur de $K_{IC}$ après un durcissement pendant > 7 jours dans l'intervalle de 0,8 à 1,2 MPam$^{1/2}$.

**13.** Système selon la revendication 1, **caractérisé en ce que** le matériau présente un changement de dimensions < 0,2 % linéaire et/ou une pression de dilatation pendant le durcissement < 3 MPa.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SE 463493
- SE 502987
- SE 519990
- SE 519991
- SE 522510
- SE 522511
- SE 522512
- SE 523671
- SE 02039105
- WO 0021489 A
- WO 0176534 A
- WO 0176535 A

**Non-patent literature cited in the description**

- Strong and safe brittle ceramics - Materials aspects of design with structural ceramics. **L. HERMANSSON.** Advanced Ceramics for Structural and Tribological Applications. 1995